(19) Europäisches Patentamt

European Patent Office

Office européen des brevets

(11) **EP 0 788 772 B1**

(12) **FASCICULE DE BREVET EUROPEEN**

(45) Date de publication et mention
de la délivrance du brevet:
**13.11.2002 Bulletin 2002/46**

(51) Int Cl.[7]: **A61B 17/16**, B23D 77/12

(21) Numéro de dépôt: **97400190.1**

(22) Date de dépôt: **28.01.1997**

(54) **Alésoir hélicoidal inverse**

Reidahle mit Linksgewinde

Reamer with left-hand helix

(84) Etats contractants désignés:
**AT CH DE ES FR GB IT LI**

(30) Priorité: **08.02.1996 FR 9601540**

(43) Date de publication de la demande:
**13.08.1997 Bulletin 1997/33**

(73) Titulaire: **Deckner, André Georges
F-75015 Paris (FR)**

(72) Inventeur: **Deckner, André Georges
F-75015 Paris (FR)**

(74) Mandataire: **Eidelsberg, Victor Albert et al
Cabinet Flechner
22, Avenue de Friedland
75008 Paris (FR)**

(56) Documents cités:
**EP-A- 0 330 107 DE-A- 2 542 056
US-A- 4 725 171 US-A- 5 527 316**

## Description

La présente invention concerne un alésoir hélicoïdal destiné notamment à donner à la cavité médullaire d'un os creux la forme que l'on souhaite.

La cavité destinée à être mise en forme conique doit préexister ou être créée au préalable avec un instrument de perçage traditionnel, car l'alésoir de la présente invention n'a pas comme fonction principale de percer un trou.

On connaît déjà un foret qui a l'inconvénient de traverser la face latérale de l'os lorsque la cavité médullaire est incurvée. Si l'on prévoit d'arrondir la pointe du foret pour prévenir toute perforation, le foret peut se bloquer et l'os se rompre.

DE 2 542 056 par exemple décrit un instrument pour percer des os.

L'invention pallie ces inconvénients par un alésoir d'une grande sécurité, qui permet de suivre la courbe d'une cavité médullaire, tout en restant centré.

L'invention a pour objet un alésoir formé d'une queue prolongée d'une tige de forme sensiblement hélicoïdale se terminant par une pointe du côté opposé à la queue et ayant une arête tournée vers la pointe caractérisé en ce que la tige affecte la forme d'une seule lame souple enroulée en hélice par rapport à un axe imaginaire, au moins la partie de l'arête tournée vers la pointe est tranchante et le sens d'enroulement de l'hélice est contraire au sens de rotation prévu normalement pour le fonctionnement de l'alésoir, la pointe comporte au moins une partie de section transversale, perpendiculaire à l'axe imaginaire, circulaire continue, et la queue comporte une noix dont au moins une partie de la section transversale est circulaire continue et a un rayon égal ou supérieur au plus grand rayon vecteur de l'hélice.

L'alésoir inverse selon l'invention a comme propriété importante de ne pas avoir tendance à progresser de lui-même dans la cavité en cours de préparation. Lorsqu'il est actionné en rotation et exerce une friction sur la cavité, il tend automatiquement à reculer. Il ne progresse que si l'opérateur exerce une forte poussée axiale, et la coupe cesse dès que cette poussée cesse. Il ne se bloque pas puisqu'il a automatiquement tendance à reculer. De plus, il est rendu souple car constitué d'une seule lame et ainsi est introduit plus facilement dans une cavité médullaire incurvée. Malgré sa souplesse, il reste bien centré par la noix et par la pointe.

Le sens habituel d'enroulement de la spirale constituant les instruments de perçage usuels peut être défini comme suit : Pour un observateur situé du côté de la queue de l'instrument et éloigné de la pointe, la spirale constitutive s'enroule dans le sens des aiguilles d'une montre, tout en s'éloignant de la queue en direction de la pointe.

L'alésoir inverse suivant la présente invention diffère essentiellement des instruments de perçage habituels en ce que la spirale constituant l'instrument s'enroule dans le sens opposé aux aiguilles d'une montre dans les conditions d'observation du paragraphe précédent.

En cours d'utilisation, le sens de rotation habituel des instruments de perçage peut être précisé comme suit : Si l'observateur est situé du côté de la queue de l'instrument et éloigné de la pointe, l'instrument tourne dans le sens des aiguilles d'une montre.

L'alésoir inverse suivant la présente invention est destiné normalement à tourner également dans le sens des aiguilles d'une montre, dans les mêmes conditions d'observation (tourne-à-droite).

Un mode de réalisation préféré de l'invention consiste à donner à la pointe de l'instrument une forme arrondie et polie. Cet arrondi est destiné à guider la pointe de l'instrument dans la cavité préexistante sans accrocher, sans endommager et surtout sans transpercer la surface intérieure rencontrée, même si celle-ci est irrégulière.

Suivant un mode de réalisation préféré de l'invention, la partie de l'alésoir qui comporte l'arête tranchante est souple.

Cette relative souplesse de l'instrument permet d'introduire ce dernier également dans des corps creux préexistants dont le canal intérieur n'a pas un axe rectiligne, mais est légèrement incurvé.

Un perfectionnement de l'invention est que la tangente en tout point de l'arête tranchante fasse avec l'axe de la tige un angle sensiblement constant dans l'espace. Le but recherché par l'obtention d'un angle sensiblement constant du tranchant avec la matière (os) à tailler est une bonne répartition de l'effet de coupe sur toute la surface à tailler.

On peut donner à l'arête tranchante sensiblement la forme d'une spirale logarithmique en 3 dimensions, qui possède simultanément la propriété d'être inscrite sur une surface conique de révolution et dont la tangente en tout point forme un angle constant dans l'espace avec l'axe.

Une forme de la spirale logarithmique plane est :

$$R = e^{k\theta}$$

où

R est le rayon-vecteur ou distance entre le "pôle asymptotique" de la courbe et le point courant de la courbe

e est la base des Logarithmes Népériens valant environ 2,71828

$\theta$ (theta) est le paramètre de la fonction et représente l'angle entre l'un des axes du plan et le rayon-vecteur, et

K la constante calculée précisant la courbe et définissant entre autres l'angle de la tangente en tout point.

Pour une spirale logarithmique en 3 dimensions, la troisième dimension, qui dans le cas de l'alésoir de la présente invention correspond à l'axe longitudinal, peut être régie par la forme :

$$Z = e^{L\theta}$$

où

Z est la projection du point courant de la courbe sur l'axe longitudinal, et
L la constante dont dépend le nombre de spires du tranchant.

L'effet recherché pour un alésoir selon l'invention, dont la conformation suit sensiblement la formulation précédente et dont les épaisseurs et sections sont calculées en conséquence, est une élasticité régulièrement progressive, plus rigide vers la queue et plus élastique vers la pointe.

Cette conformation vise une adaptation optimale à la courbure d'une cavité osseuse, en prévision de l'implantation d'une tige prothétique également incurvée, et en particulier incurvée selon une spirale logarithmique appropriée.

Les propriétés décrites précédemment permettent de rechercher pour la présente invention, l'obtention d'une cavité alésée ayant la conformation d'un tronc de cône dont l'axe longitudinal serait, non pas rectiligne, mais curviligne, permettant un autoblocage dans cette cavité d'un implant possédant également une forme partiellement tronconique, incurvée sur un axe curviligne approprié.

D'une manière générale, il est bon que la surface-enveloppe imaginaire de la tige s'évase de la pointe vers la queue, ce qui contribue à empêcher que la prothèse ne s'enfonce trop dans la cavité médullaire après sa mise en place.

Suivant une variante, le pas hélicoïdal de l'arête tranchante est constant.

Suivant une autre variante, la pointe a des arêtes tranchantes permettant à l'alésoir de créer lui-même le trou d'entrée dans l'os et de réaliser un alésage conique en une seule opération.

L'invention vise aussi un procédé de conformation de la cavité médullaire d'un os creux qui consiste à faire tourner dans la cavité un alésoir suivant l'invention.

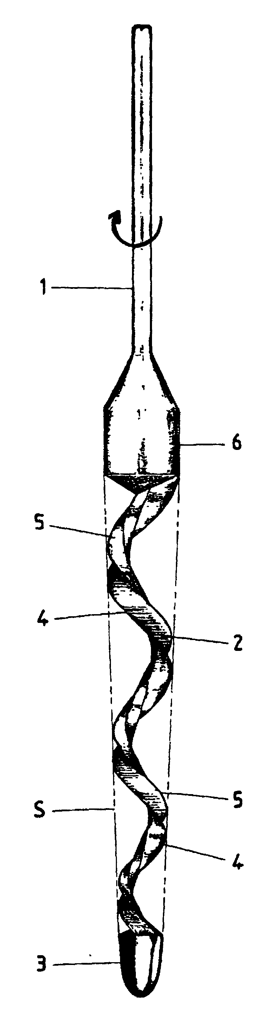

Au dessin annexé, donné uniquement à titre d'exemple, la figure 1 est une vue en perspective d'un alésoir suivant l'invention dans une version utilisable dans le domaine de la chirurgie osseuse, pour préparer le lit osseux d'un os endommagé de manière à pouvoir ancrer profondément, dans une zone osseuse encore saine, une prothèse,

L'alésoir est constitué d'une queue (1) destinée à la fixation, par tout moyen approprié, sur une machine tournante, et d'une tige (2) métallique issue de la queue (1). La tige (2) est une lame souple torsadée en hélice.

La lame comprend deux arêtes (4 et 5), l'une (4) dénommée arête avant qui est tournée vers la pointe (3) de la lame, l'autre (5) dénommée arête arrière qui est tournée vers la queue (1). Toute l'arête (4) est tranchante, tandis que l'arête (5) n'est pas tranchante. La flèche indique le sens de rotation prévu normalement pour le fonctionnement de l'alésoir (tourne-à-droite). L'hélice qui constitue la tige (2) tourne à gauche en sens inverse du sens de rotation indiqué par la flèche. Le pas de l'hélice est constant.

La pointe (3) est arrondie ce qui, de pair avec la structure de la lame, contribue à ce que la pointe (3) de la lame ne puisse pas percer le canal du corps creux dans lequel l'alésoir est introduit. La pointe (3) comporte une partie de section transversale, perpendiculaire à l'axe imaginaire de l'alésoir, qui passe par l'axe de la queue (1), est circulaire continue et la queue a une noix (6) de rayon égal ou supérieur au plus grand rayon vecteur de l'hélice

L'hélice, dans la figure 1, a un pas croissant de la pointe (3) à la queue (1).

La courbe suivie par l'arête avant (4) tranchante, est une spirale logarithmique. La surface-enveloppe (S) imaginaire de la tige (2) est tronconique en s'évasant de la pointe (3) vers la queue (1).

La pointe (3) comporte deux arêtes tranchantes.

L'alésoir peut être en métal ou en céramique.

## Revendications

**1.** Alésoir formé d'une queue (1) prolongée d'une tige (2) de forme sensiblement hélicoïdale se terminant par une pointe (3) du côté opposé à la queue (1) et ayant une arête tournée vers la pointe **caractérisé en ce que**

la tige (2) affecte la forme d'une seule lame souple enroulée en hélice par rapport à un axe imaginaire,
au moins la partie de l'arête (4) tournée vers la pointe est tranchante et le sens d'enroulement de l'hélice est contraire au sens de rotation prévu normalement pour le fonctionnement de l'alésoir,
la pointe (3) comporte au moins une partie de section transversale, perpendiculaire à l'axe imaginaire, circulaire continue, et
la queue (1) comporte une noix (6) dont au moins une partie de la section transversale, est circulaire continue et a un rayon égal ou supérieur au plus grand rayon vecteur de l'hélice.

**2.** Alésoir suivant la revendication 1, **caractérisé en**

**ce que** toute l'arête (4) tournée vers la pointe est tranchante.

3. Alésoir selon l'une des revendications précédentes, **caractérisé en ce que** la surface-enveloppe (S) imaginaire de la tige (2) s'évase de la pointe (3) vers la queue (1).

4. Alésoir selon la revendication 3, **caractérisé en ce que** la surface-enveloppe (S) est un tronc de cône.

5. Alésoir selon l'une des revendications précédentes, **caractérisé en ce que** la tangente en tout point de l'arête (4) tranchante fait avec l'axe (XX') de la tige (2) un angle dans l'espace qui est constant.

6. Alésoir selon les revendications 4 et 5, **caractérisé en ce que** l'arête (4) tranchante de la tige (2) décrit une spirale logarithmique en 3 dimensions.

7. Alésoir selon l'une des revendications 1 à 4, **caractérisé en ce que** le pas hélicoïdal de l'arête (4) tranchante est sensiblement constant.

8. Alésoir selon l'une des revendications 1 à 7, **caractérisé en ce que** la pointe (3) est arrondie ou comporte des arêtes tranchantes.

9. L'utilisation d'un alésoir suivant l'une des revendications précédentes pour conformer la cavité médullaire d'un os creux.

**Patentansprüche**

1. Reibahle, bestehend aus einem Endstück (1), welches durch eine im Wesentlichen spiralförmige Stange (2) verlängert ist, und die auf der entgegengesetzten Seite des Endstücks (1) in eine Spitze (3) mündet und eine Kante hat, die zu der Spitze gewandt ist,
   **dadurch gekennzeichnet, dass**
   die Stange (2) die Form einer einzelnen federnden Klinge aufweist, welche zu einer Spirale gegenüber einer imaginären Achse gedreht ist,
   mindestens der Teil der Kante (4) scharf ist, der zu der Spitze hin gedreht ist, und die Drehrichtung der Spirale entgegengesetzt zu der normalerweise für den Betrieb der Reibahle vorgesehenen Rotationsrichtung ist,
   die Spitze (3) mindestens einen zur imaginären Achse senkrechten und gleichmäßig runden Querschnittsteil beinhaltet und
   das Endstück (1) ein Basisteil (6) beinhaltet, von welchem mindestens ein Querschnittsteil gleichmäßig rund ist und einen gleichen oder größeren Radius als der größte Vektorradius der Spirale hat.

2. Reibahle gemäß Anspruch 1,
   **dadurch gekennzeichnet, dass** die gesamte zur Spitze gerichtete Kante (4) scharf ist.

3. Reibahle gemäß einem der vorangegangenen Ansprüche,
   **dadurch gekennzeichnet, dass** die imaginäre Hüllfläche (S) der Stange (2) sich von der Spitze (3) zum Endstück (1) konisch erweitert.

4. Reibahle gemäß Anspruch 3,
   **dadurch gekennzeichnet, dass** die Hüllfläche (S) ein Kegelstumpf ist.

5. Reibahle gemäß einem der vorangegangenen Ansprüche,
   **dadurch gekennzeichnet, dass** die Tangente in allen Punkten der scharfen Kante (4) mit der Achse (XX') der Stange (2) einen Winkel im Raum bildet, der konstant ist.

6. Reibahle gemäß den Ansprüchen 4 und 5,
   **dadurch gekennzeichnet, dass** die scharfe Kante (4) der Stange (2) eine logarithmische dreidimensionale Spirale bildet.

7. Reibahle gemäß einem der Ansprüche 1 bis 4,
   **dadurch gekennzeichnet, dass** die spiralförmige Steigung der scharfen Kante (4) im Wesentlichen konstant ist.

8. Reibahle gemäß einem der Ansprüche 1 bis 7,
   **dadurch gekennzeichnet, dass** die Spitze (3) abgerundet ist oder scharfe Kanten beinhaltet.

9. Verwendung einer Reibahle gemäß einem der vorangegangenen Ansprüche, um die Knochenmarkshöhle eines hohlen Knochens anzupassen.

**Claims**

1. Reamer formed of a tang (1) extended by a shank (2) having a substantially helical shape ending in a point (3) on the opposite side to the tang (1) and having an edge turned towards the point, **characterized in that**
   the shank (2) takes the form of a single flexible blade wound into a helix about an imaginary axis,
   at least the part of the edge (4) turned towards the point is cutting and the direction in which the helix is wound is opposite to the direction of rotation normally provided for operating the reamer,
   the point (3) comprises at least one part having a continuously circular cross-section perpendicular to the imaginary axis, and
   the tang comprises a boss (6), of which at least part of the transverse section is continuously circular and has a radius equal to or greater than the largest radius vector of the helix.

2. Reamer according to claim 1, **characterized in that** the edge (4) turned towards the point is cutting.

3. Reamer according to one of the preceding claims, **characterized in that** the imaginary enveloping surface (S) of the shank (2) widens out from the point (3) towards the tang (1).

4. Reamer according to claim 3, **characterized in that** the enveloping surface (S) is a truncated cone.

5. Reamer according to one of the preceding claims, **characterized in that** the tangent at any point of the cutting edge (4) makes a constant angle in space with the axis (XX') of the shank (2).

6. Reamer according to claims 4 and 5, **characterized in that** the cutting edge (4) of the shank (2) describes a three-dimensional logarithmic spiral.

7. Reamer according to one of claims 1 - 4, **characterized in that** the helical pitch of the cutting edge (4) is substantially constant.

8. Reamer according to one of claims 1 - 7, **characterized in that** the point (3) is rounded or comprises cutting edges.

9. The use of a reamer according to the preceding claims for giving the desired shape to the medullar cavity of a hollow bone.